# FASCICULE DE BREVET EUROPEEN

(11) **EP 3 297 732 B1**
(45) Date de publication et mention de la délivrance du brevet: **06.03.2019**
(21) Numéro de dépôt: 16726362.3
(22) Date de dépôt: 10.05.2016
(51) Int. Cl.: A61Q 15/00, A61K 8/19, A61K 8/25, A61K 8/34, A61K 8/39, A61K 8/73, A61K 8/92

(54) **COMPOSITION DEODORANTE ANHYDRE A BASE DE BICARBONATE**
WASSERFREIE DESODORIERENDE ZUSAMMENSETZUNG AUS BICARBONAT
ANHYDROUS DEODORANT COMPOSITION MADE FROM BICARBONATE

(30) Priorité: 21.05.2015 FR 1554552
(43) Date de publication de la demande: 28.03.2018
(73) Titulaire: LABORATOIRES M&L, 04100 Manosque (FR)
(72) Inventeur: MILLET, Magali, 04190 Les Mees (FR); SALLES, Florian, 04100 Manosque (FR)
(74) Mandataire: Cabinet Becker et Associés
(86) Numéro de dépôt international: PCT/FR2016/051095
(87) Numéro de publication internationale: WO 2016/185113

(56) Documents cités:
- CA-A1- 2 808 807
- US-A- 5 376 363

## Description

### OBJET DE L'INVENTION

La présente invention concerne une composition cosmétique anhydre à base de sel de bicarbonate, ainsi que son utilisation cosmétique en tant que déodorant. Elle concerne également un produit déodorant renfermant cette composition.

### ARRIERE-PLAN DE L'INVENTION

Les déodorants et antiperspirants sont devenus des produits d'hygiène aussi incontournables que les shampooings et les dentifrices. Si les premiers agissent sur les odeurs de transpiration soit en les camouflant, soit en ciblant les bactéries se nourrissant de la sueur apocrine, soit encore en absorbant la transpiration, les seconds régulent la quantité de sueur émise. L'innocuité des sels d'aluminium utilisés comme agents anti-transpirants a été remise en cause ces dernières années, ce qui a conduit les consommateurs à se tourner vers des produits renfermant des agents déodorants naturels tels que le talc et/ou certaines plantes sous forme séchée ou sous forme d'huiles essentielles. L'efficacité de ces produits n'est toutefois pas toujours optimale.

Le bicarbonate de sodium constitue un actif déodorant naturel reconnu. Aux doses considérées comme efficaces, sa formulation est en revanche compliquée par son incompatibilité avec de nombreuses matières premières, en particulier lorsqu'il doit être formulé dans des compositions anhydres solides ou semi-solides présentant de bonnes propriétés cosmétiques.

### RESUME DE L'INVENTION

Après de nombreuses recherches, la Demanderesse a mis au point une composition dans laquelle des quantités efficaces de sel de bicarbonate peuvent être associées à une quantité importante d'huiles sans nuire à la stabilité de la composition. Il est ainsi possible de formuler des déodorants anhydres sous forme de crème ou de sticks de texture souple, à base d'ingrédients d'origine naturelle. Ces compositions se sont avérées faciles d'application, efficaces contre les odeurs de transpiration et capables de déposer sur la peau un film doux, non gras et non collant.

L'invention a ainsi pour objet une composition cosmétique anhydre renfermant :
(a) de 1 à 20% en poids d'au moins un sel de bicarbonate,
(b) de 20 à 50% en poids d'au moins une huile,
(c) au moins un structurant de phase grasse,
(d) de la glycérine, et
(e) au moins un ester de polyglycérol et d'un acide gras renfermant de 12 à 30 atomes de carbone.

Elle a également pour objet l'utilisation cosmétique de cette composition pour traiter les odeurs corporelles humaines, en particulier les odeurs axillaires.

Elle concerne enfin un produit déodorant sous forme de tube, de pot ou de tout autre conditionnement, notamment de forme oblongue, adapté à distribuer une composition semi-solide, contenant la composition selon l'invention sous forme de crème, ou sous forme de conditionnement adapté à distribuer un bâton, contenant la composition selon l'invention sous forme de bâton.

### DESCRIPTION DETAILLEE

La composition selon l'invention est une composition anhydre, en ce sens qu'elle renferme moins de 5% en poids d'eau, avantageusement moins de 1% en poids d'eau, qui peut être apportée uniquement par ses ingrédients constitutifs. On préfère qu'elle ne renferme pas d'eau.

Elle comprend un sel de bicarbonate, en tant qu'agent déodorant. Celui-ci peut représenter de 1 à 20 % en poids, et de préférence de 5 à 15% en poids, par rapport au poids total de la composition. Comme sels de bicarbonate, on peut citer les sels de sodium, de potassium, de magnésium et d'ammonium, le sel de sodium étant préféré pour une utilisation dans cette invention. Selon un mode de réalisation, la composition ne renferme pas de sels d'aluminium. En revanche, elle peut toutefois contenir au moins un actif déodorant additionnel choisi parmi : les agents bactériostatiques ou bactéricides, tels que la chlorhexidine et ses sels ; le triclosan ; le triclocarban ; le farnesol ; les huiles essentielles d'origine végétale, choisies par exemple parmi les huiles essentielles d'origan, de palmarosa, de menthe poivrée, de lavande, de citron et d'arbre à thé ; les extraits végétaux tels que les extraits de graines de pamplemousse ; les sels de zinc tels que le gluconate, le pidolate et le ricinoléate de zinc ; et leurs mélanges.

Comme indiqué précédemment, il a été observé que la composition selon l'invention était stable en présence de bicarbonate, quand bien même elle renferme de grandes quantités d'huile, à savoir de 20 à 50% en poids, et notamment de 25 à 40% en poids, d'une ou plusieurs huiles, par rapport au poids total de la composition. Au sens de la présente invention, on entend par "huile" un composé liquide à température ambiante (25°C) et pression atmosphérique (105 Pa) qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau. Les huiles peuvent être volatiles ou non volatiles. Par "huile non volatile", on entend dans cette description une huile restant sur la peau à 25°C et pression atmosphérique pendant au moins une heure, en l'absence de frottement, et/ou ayant une pression de vapeur inférieure à 0,001 mm Hg dans ces conditions. Les huiles incluses dans la composition selon l'invention peuvent ou non être volatiles ; elles sont avantageusement non volatiles. En variante, il peut s'agit d'un mélange d'huiles non volatiles (majoritaires en poids) et volatiles (minoritaires en poids). Des exemples d'huiles volatiles sont notamment les alcanes linéaires en C11 à C14. En outre, on préfère que les huiles non volatiles soient choisies parmi les huiles hydrocarbonées, c'est-à-dire qu'elles renferment exclusivement des atomes de carbone, d'hydrogène et éventuellement d'oxygène.

Des exemples d'huiles non volatiles comprennent :
- les esters d'acides et de mono-alcool choisis parmi : les mono- et polyesters d'acides linéaires saturés en C2-C10 (de préférence en C6-C10) et de mono-alcools linéaires saturés en C10-C18 (de préférence C10-C14), les mono- et polyesters d'acides linéaires saturés en C10-C20 et de mono-alcools ramifiés ou insaturés en C3-C20 (de préférence C3-C10) ; les mono- et polyesters d'acides ramifiés ou insaturés en C5-C20 et de mono-alcools ramifiés ou insaturés en C5-C20 ; les mono- et polyesters d'acides ramifiés ou insaturés en C5-C20 et de mono-alcools linéaires en C2-C4 ;
- les triglycérides d'acides gras en C6-C12, tels que les triglycérides d'acides caprylique et caprique et la triheptanoïne ;
- les acides gras ramifiés et/ou insaturés en C10-C20 (tels que les acides linoléique, laurique et myristique) ;
- les alcools gras ramifiés et/ou insaturés en C10-C20 (tels que l'octyldodécanol et l'alcool oléylique) ;
- les hydrocarbures tels que le squalane végétal extrait de l'huile d'olive ;
- les carbonates de dialkyle, tels que le dicaprylyl carbonate et le diéthylhexyl carbonate ;
- les dialkyléthers tels que le dicaprylyl éther ; et
- leurs mélanges.
On peut également citer les huiles végétales qui contiennent un ou plusieurs des constituants précités.

Comme esters d'acides et de monoalcools qui constituent la classe préférée d'huiles non volatiles selon l'invention, on peut notamment citer les monoesters tels que le mélange de caprate et caprylate de coco, le macadamiate d'éthyle, l'ester éthylique de beurre de karité, l'isostéarate d'isostéaryle, l'isononanoate d'isononyle, l'isononanoate d'éthylhexyle, le néopentanoate d'hexyle, le néopentanoate d'éthylhexyle, le néopentanoate d'isodécyle, le néopentanoate d'isostéaryle, le néopentanoate d'isodécyle, le myristate d'isopropyle, le myristate d'octyldodécyle, le palmitate d'isopropyle, le palmitate d'éthylhexyle, le laurate d'hexyle, le laurate d'isoamyle, le nonanoate de cétostéaryle, le capylate de propylheptyle et leurs mélanges. D'autres esters utilisables sont les diesters d'acides et de monoalcools tels que l'adipate de disopropyle, l'adipate de diéthylhexyle, le sébaçate de diisopropyle et le sébaçate de diisoamyle.

Des exemples d'huiles végétales sont notamment les huiles de germe de blé, de tournesol, d'argan, d'hibiscus, de coriandre, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de lavande, de bourrache, de millet, d'orge, de quinoa, de seigle, de carthame, de bancoulier, de passiflore, de rosier muscat ou de camélia.

Pour obtenir une composition stable présentant de bonnes propriétés cosmétiques, la composition selon l'invention renferme en outre une combinaison d'au moins un structurant de phase grasse avec de la glycérine et au moins un ester de polyglycérol et d'alcool gras renfermant de 12 à 30 atomes de carbone.

Par "structurant de phase grasse", on entend un composé capable d'épaissir les huiles contenues dans la composition, choisi notamment parmi les cires, les gélifiants de phase grasse et les corps gras pâteux, ainsi que leurs mélanges.

Le terme "cire" désigne, dans le contexte de cette description, un corps gras solide à 25°C, à changement d'état solide / liquide réversible, ayant un point de fusion généralement compris entre 30 et 160°C, de préférence entre 50 et 90°C, tel que mesuré par DSC. Des exemples de cires sont en particulier les cires d'origine animale ou végétale, telles que les cires d'abeille, d'insecte de Chine, de candelilla, de carnauba ou d'acacia ; les huiles végétales hydrogénées et éventuellement modifiées par l'acide isostéarique, notamment les huiles de colza, de soja, de tournesol, de jojoba, de coprah et de ricin hydrogénées ; les esters d'acides gras linéaires saturés en C14-C30 et d'alcools gras linéaires saturés en C16-C36 ; les acides linéaires et saturés en C10-C30 ; les alcools linéaires et saturés en C8-C30 ; et leurs mélanges. Ces cires peuvent se trouver sous forme micronisée, c'est-à-dire sous la forme d'une poudre dont les particules présentent une taille moyenne en nombre inférieure ou égale à 50 µm, et notamment allant de 0,5 à 50 µm, de préférence allant de 1 à 30 µm, voire allant de 3 à 20 µm, où la « taille moyenne en nombre » correspond à la dimension donnée par la distribution granulométrique statistique à la moitié de la population, dite D50.

Par "gélifiants de phase grasse", il est fait référence aux composés modifiant la rhéologie de la phase grasse par formation d'un réseau tridimensionnel. Comme composés de ce type, on peut notamment citer les argiles (en particulier les bentonites et les hectorites) modifiées hydrophobes, notamment par du chlorure de di-stéaryl diméthyl ammonium ; les silices pyrogénées modifiées hydrophobes ; le palmitate et le myristate de dextrine ; les polyamides, les copolymères oléfine(s) / styrène, les poly(acrylates d'alkyle) ; les glycérides d'acides gras (de préférence linéaires et saturés) en C₁₆-C₂₆ tels que le composé Nomcort® HKG ; les dérivés de cellulose et les mélanges en contenant ; et leurs mélanges. Certaines huiles végétales hydrogénées peuvent également être considérées comme des gélifiants de phase grasse.

Enfin, les corps gras pâteux utilisables comme structurants de phase grasse sont définis comme des corps gras à changement d'état liquide / solide réversible, présentant à l'état solide une organisation cristalline anisotrope et comportant à une température de 23°C une fraction liquide et une fraction solide. On préfère utiliser les beurres végétaux. Les beurres de karité, de cacao et de mangue constituent des exemples de tels corps gras pâteux.

Selon une forme d'exécution préférée, le structurant de phase grasse inclus dans la composition selon l'invention est constitué d'au moins un ingrédient choisi parmi les cires d'origine animale, les cires d'origine végétale, les gélifiants de phase grasse et leurs mélanges, éventuellement combiné à au moins un beurre végétal. Plus préférentiellement encore, le structurant de phase grasse est constitué d'une association d'au moins une cire animale et/ou végétale avec au moins un gélifiant de phase grasse.

Ces structurants de phase grasse peuvent représenter de 5 à 40%, et de préférence de 15 à 30% du poids total de la composition.

Un autre constituant de la composition selon l'invention, contribuant à sa stabilité, est la glycérine. Celle-ci peut représenter de 1 à 15% et de préférence de 3 à 10% du poids de la composition.

La composition selon l'invention contient également au moins un ester de polyglycérol et d'un acide gras renfermant de 12 à 30 et de préférence de 18 à 22 atomes de carbone. L'acide gras peut être choisi parmi les acides linéaires saturés, les acides ramifiés saturés, les acides linéaires mono-insaturés et leurs mélanges. Ces acides peuvent éventuellement être mono- ou polyhydroxylés. Des exemples de tels composés sont notamment les acides stéarique, isostéarique, caprique, arachidique, béhénique, hydroxypalmitique, hydroxystéarique, oléique et érucique. Le polyglycérol peut être obtenu par condensation de deux à six motifs glycérol. Il s'agit de préférence de polyglycérol-3 ou -4. Le polyglycérol peut être partiellement ou totalement estérifié pour obtenir l'ester utilisé dans la présente invention. Il peut notamment être le produit de l'estérification d'une cire à l'aide de polyglycérol. Un produit de ce type est disponible dans le commerce auprès de la société GATTEFOSSE sous la dénomination commerciale Acticire®.

Cet ester peut représenter de 0,1 à 5% et de préférence de 0,5 à 3% du poids total de la composition.

La composition selon l'invention renferme en outre avantageusement une ou plusieurs charges pulvérulentes, qui sont adaptées à absorber l'humidité et la sueur et qui se présentent généralement sous forme de microparticules poreuses ou creuses, de préférence poreuses. Ces microparticules sont en principe sensiblement sphériques. Ces charges peuvent notamment être choisies parmi :
- les charges organiques telles que : les poudres de polysaccharides et en particulier d'amidon natif, d'amidon modifié et de cellulose ; les poudres de polymères acryliques tels que le poly(méthacrylate de méthyle), de polyamides ou de polyoléfines ; les poudres d'algues séchées telles que Corallina officinalis ;
- les charges inorganiques telles que la silice, les argiles, la perlite et le talc ;
- et leurs mélanges.

Les amidons sont de préférence choisis parmi l'amidon de maïs, de riz, de tapioca ou de blé. Les amidons modifiés constituent des charges organiques préférées pour une utilisation dans cette invention. Des exemples d'amidons modifiés sont les amidons éventuellement pré-gélatinisés et/ou oxydés, qui sont estérifiés par un anhydride alkénylsuccinique, notamment par l'anhydride octénylsuccinique ou dodécylsuccinique, éventuellement en présence de chlorure de calcium, ainsi que les amidons éthérifiés, notamment hydroxypropylés ou carboxyméthylés, et les amidons cationiques, notamment quaternisés. On peut également citer l'amidon réticulé par le trimétaphosphate de sodium.. Comme charge inorganique, on préfère utiliser la silice.

Ces charges peuvent représenter de 10 à 30% en poids, et de préférence de 15 à 30% en poids, par rapport au poids total de la composition.

Elle peut en outre comprendre des additifs choisis notamment parmi des parfums, des anti-oxydants tels que le tocophérol, des colorants, des conservateurs et leurs mélanges.

On préfère que la composition selon l'invention renferme une quantité d'au moins 90% en poids d'ingrédients d'origine végétale, telle que déterminée selon la norme ASTM D7026.

La composition décrite précédemment peut être utilisée comme produit déodorant, qui peut se présenter sous forme de crème (ayant avantageusement la texture d'un baume) ou de stick présentant avantageusement une texture souple.

### EXEMPLES

L'invention sera mieux comprise à la lumière des exemples suivants, qui sont donnés à titre purement illustratif et n'ont pas pour but de limiter la portée de l'invention, définie par les revendications annexées.

### Exemple 1 : Baume déodorant

On a préparé un baume par mélange des ingrédients, et dans les proportions pondérales, indiqués ci-dessous.

| | |
|---|---|
| Beurres végétaux | 20,00% |
| Cires végétales et ester* | 9,00% |
| Huiles végétales | 24,00% |
| Glycérine | 6,00% |
| Bicarbonate de sodium | 10,00% |
| Amidon modifié | 23,00% |
| Silice | 2,00% |
| Huiles essentielles | qs |
| Anti-oxydant | qs |
| Parfum | qs |
| Total : | 100,00% |

| | |
|---|---|
| *dont Acticire® de GATTEFOSSE, renfermant des esters d'acides gras en C12-C30 de polyglycérol-3. | |

Ce baume peut être conditionné dans des pots et prélevé à l'aide d'une spatule.

### Exemple 2 : Baume déodorant

| | |
|---|---|
| Beurres végétaux | 20,00% |
| Cires végétales et ester* | 9,00% |
| Huiles végétales | 33,00% |
| Glycérine | 6,00% |
| Bicarbonate de sodium | 10,00% |
| Amidons natifs | 20,00% |
| Anti-oxydant | 0,20% |
| Parfum | 1,50% |

| | |
|---|---|
| *dont Acticire® de GATTEFOSSE, renfermant des esters d'acides gras en C12-C30 de polyglycérol-3. | |

### Exemple 3 : Test de stabilité

On a prélevé quatre échantillons A à D de la composition de l'Exemple 1, qui ont été répartis dans quatre piluliers stockés respectivement à 4°C, 25°C, 40°C et 50°C. La stabilité des échantillons A à C est évaluée chaque semaine pendant 1 mois puis tous les 15 jours pendant 2 mois et celle de l'échantillon D est évaluée chaque semaine pendant 1 mois.

A titre comparatif, on a évalué la stabilité d'un produit similaire, renfermant : un beurre végétal, des huiles végétales, de la glycérine, du bicarbonate de sodium, de l'amidon, des huiles essentielles et un anti-oxydant. Ce produit ne renfermait pas d'ester de polyglycérol, de cire, ni de gélifiant de phase grasse Il a également été réparti en quatre échantillons, comme décrit précédemment.

On a observé l'aspect des échantillons testés. Les résultats sont présentés dans le tableau ci-dessous :

| Produit testé | Après 3 mois à 4°C | Après 3 mois à 25°C | Après 3 mois à 40°C | Après 1 mois à 50°C |
|---|---|---|---|---|
| Exemple 1 | stable | stable | stable | stable |
| Comparatif | stable | stable | aspect granuleux ; exsudation dès 1 semaine et couleur plus foncée | déphasage dès 1 semaine et brunissement |

Il ressort de ce test que le produit selon l'invention est plus stable à 40°C, c'est-à-dire dans des conditions de vieillissement accéléré qui sont considérées comme représentatives du comportement du produit stocké pendant trois ans dans des conditions normales de conservation.

### Exemple 4 : Analyse sensorielle

Un panel de 19 volontaires a été recruté pour évaluer le baume de l'Exemple 1. Le produit a été appliqué sous les aisselles à la spatule, suivi d'un massage pour le faire pénétrer dans la peau.
Les panélistes ont majoritairement considéré que le produit était facile d'application (17/19), limitait la formation d'odeurs de transpiration (17/19) et assurait une bonne protection déodorante (16/19), sans laisser de traces sur les vêtements (15/19).

### Exemple 5 : Analyse sensorielle - Essai comparatif

Le baume de l'Exemple 2 a été comparé à un produit similaire, renfermant : un beurre végétal, des huiles végétales, de la glycérine, du bicarbonate de sodium, de l'amidon, des huiles essentielles et un anti-oxydant. Ce produit ne renfermait pas d'ester de polyglycérol, de cire, ni de gélifiant de phase grasse.
Pour ce faire, 9 volontaires ont été recrutés. Le matin du test, il leur a été demandé de se laver les aisselles avec un savon neutre avant d'appliquer, à l'aide d'une spatule, de 0,4 à 0,5g du baume de l'Exemple 2 sous une aisselle et la même quantité du baume comparatif sous l'autre aisselle. Les panélistes ont ensuite revêtu un tee-shirt en Lycra noir, préalablement lavé à l'aide d'une lessive neutre, qu'ils ont conservé toute la journée. L'efficacité des baumes a été auto-évaluée après 8 heures, au laboratoire de test.

Le baume de l'Exemple 2 était plus efficace que le baume comparatif contre les odeurs de transpiration après 8h (pas d'odeur pour 8 panélistes contre 6 panélistes). Il générait en outre moins de traces sur le tee-shirt (pas de traces pour 5 panélistes contre 3 panélistes). En outre, les panélistes ont préféré la texture plus souple du baume de l'Exemple 2.

## Revendications

1. Composition cosmétique anhydre renfermant :
(a) de 1 à 20% en poids d'au moins un sel de bicarbonate,
(b) de 20 à 50% en poids d'au moins une huile,
(c) au moins un structurant de phase grasse,
(d) de la glycérine, et
(e) au moins un ester de polyglycérol et d'un acide gras renfermant de 12 à 30 atomes de carbone.

2. Composition selon la revendication 1, **caractérisée en ce que** le sel de bicarbonate représente de 5 à 15% en poids, par rapport au poids total de la composition.

3. Composition selon l'une quelconque des revendications 1 ou 2, **caractérisée en ce que** le structurant de phase grasse est constitué d'au moins un ingrédient choisi parmi les cires d'origine animale, les cires d'origine végétale, les gélifiants de phase grasse et leurs mélanges, éventuellement combiné à au moins un beurre végétal, plus préférentiellement le structurant de phase grasse est constitué d'une association d'au moins une cire animale et/ou végétale avec au moins un gélifiant de phase grasse.

4. Composition selon l'une quelconque des revendications 1 à 3, **caractérisée en ce que** la glycérine représente de 1 à 15% en poids, de préférence de 3 à 10% en poids, par rapport au poids total de la composition.

5. Composition selon l'une quelconque des revendications 1 à 4, **caractérisée en ce qu'**elle renferme en outre une ou plusieurs charges pulvérulentes sous forme de microparticules poreuses ou creuses, choisies de préférence parmi les amidons modifiés, la silice et leurs mélanges.

6. Composition selon la revendication 5, **caractérisée en ce que** les charges pulvérulentes représentent de 10 à 30% en poids, et de préférence de 15 à 30% en poids, par rapport au poids total de la composition.

7. Utilisation cosmétique de la composition selon l'une quelconque des revendications 1 à 6 pour traiter les odeurs corporelles humaines, en particulier les odeurs axillaires.

8. Produit déodorant sous forme de tube, de pot ou de tout autre conditionnement, notamment de forme oblongue, adapté à distribuer une composition semi-solide, contenant la composition selon l'une quelconque des revendications 1 à 6 sous forme de crème.

9. Produit déodorant sous forme de conditionnement adapté à distribuer un bâton, contenant la composition selon l'une quelconque des revendications 1 à 6 sous forme de bâton.

## Patentansprüche

1. Wasserfreie kosmetische Zusammensetzung enthaltend:
(a) 1 bis 20 Gew.-% wenigstens eines Bicarbonatsalzes,
(b) 20 bis 50 Gew.-% wenigstens eines Öls,
(c) wenigstens ein Strukturierungsmittel der Fettphase,
(d) Glycerin, und
(e) wenigstens einen Ester von Polyglycerol und einer Fettsäure, enthaltend 12 bis 30 Kohlenstoffatome.

2. Zusammensetzung gemäß Anspruch 1, **dadurch gekennzeichnet, dass** das Bicarbonatsalz 5 bis 15 Gew.-% bezogen auf das Gesamtgewicht der Zusammensetzung darstellt.

3. Zusammensetzung gemäß einem der Ansprüche 1 oder 2, **dadurch gekennzeichnet, dass** das Strukturierungsmittel der Fettphase von wenigstens einem Bestandteil gebildet ist, ausgewählt aus Wachsen tierischen Ursprungs, Wachsen pflanzlichen Ursprungs, Gelbildnern der Fettphase und ihren Gemischen, gegebenenfalls kombiniert mit wenigstens einem pflanzlichen Butter, bevorzugter das Strukturierungsmittel der Fettphase von einer Kombination aus wenigstens einem tierischen und/oder pflanzlichen Wachs mit wenigstens einem Gelbildner der Fettphase gebildet ist.

4. Zusammensetzung gemäß einem der Ansprüche 1 bis 3, **dadurch gekennzeichnet, dass** das Glycerin 1 bis 15 Gew.-%, bevorzugt 3 bis 10 Gew.-%, bezogen auf das Gesamtgewicht der Zusammensetzung darstellt.

5. Zusammensetzung gemäß einem der Ansprüche 1 bis 4, **dadurch gekennzeichnet, dass** sie außerdem einen oder mehrere pulverförmige Füllstoffe in Form von porösen oder hohlen Mikropartikeln enthält, bevorzugt ausgewählt aus modifizierten Stärken, Kieselsäure und ihren Gemischen.

6. Zusammensetzung gemäß Anspruch 5, **dadurch gekennzeichnet, dass** die pulverförmigen Füllstoffe 10 bis 30 Gew.-% und bevorzugt 15 bis 30 Gew.-% bezogen auf das Gesamtgewicht der Zusammensetzung darstellen.

7. Kosmetische Verwendung der Zusammensetzung gemäß einem der Ansprüche 1 bis 6 zur Behandlung menschlicher Körpergerüche, insbesondere Achselgerüche.

8. Desodorierendes Produkt in Form einer Tube, eines Tiegels oder einer beliebig anderen Verpackung, insbesondere in länglicher Form, die für eine Verteilung einer halbfesten Zusammensetzung geeignet ist und die Zusammensetzung gemäß einem der Ansprüche 1 bis 6 als Creme enthält.

9. Desodorierendes Produkt in Form einer Verpackung, die für eine Verteilung eines Stifts geeignet ist und die Zusammensetzung gemäß einem der Ansprüche 1 bis 6 in Form eines Stifts enthält.

## Claims

1. An anhydrous cosmetic composition containing:
(a) from 1% to 20% by weight of at least one bicarbonate salt,
(b) from 20% to 50% by weight of at least one oil,
(c) at least one fatty-phase structuring agent,
(d) glycerol, and
(e) at least one polyglycerol ester of a fatty acid containing from 12 to 30 carbon atoms.

2. The composition as claimed in claim 1, **characterized in that** the bicarbonate salt represents from 5% to 15% by weight, relative to the total weight of the composition.

3. The composition as claimed in either one of claims 1 and 2, **characterized in that** the fatty-phase structuring agent consists of at least one ingredient chosen from waxes of animal origin, waxes of plant origin, fatty-phase gelling agents and mixtures thereof, optionally combined with at least one plant butter, more preferentially the fatty-phase structuring agent consists of a combination of at least one animal and/or plant wax with at least one fatty-phase gelling agent.

4. The composition as claimed in any one of claims 1 to 3, **characterized in that** the glycerol represents from 1% to 15% by weight, preferably from 3% to 10% by weight, relative to the total weight of the composition.

5. The composition as claimed in any one of claims 1 to 4, **characterized in that** it additionally contains one or more pulverulent fillers in the form of porous or hollow microparticles, preferably chosen from modified starches, silica and mixtures thereof.

6. The composition as claimed in claim 5, **characterized in that** the pulverulent fillers represent from 10% to 30% by weight, and preferably from 15% to 30% by weight, relative to the total weight of the composition.

7. The cosmetic use of the composition as claimed in any one of claims 1 to 6 for treating human body odors, in particular axillary odors.

8. A deodorant product in the form of a tube, pot or any other packaging, in particular of oblong shape, suitable for dispensing a semi-solid composition, containing the composition as claimed in any one of claims 1 to 6 in cream form.

9. A deodorant product in a packaging form suitable for dispensing a stick, containing the composition as claimed in any one of claims 1 to 6 in stick form.
